# EUROPEAN PATENT SPECIFICATION

(11) **EP 1 870 052 B1**
(45) Date of publication and mention of the grant of the patent: **25.02.2009**
(21) Application number: 07012095.1
(22) Date of filing: 20.06.2007
(51) Int. Cl.: A61B 18/14

(54) **High frequency operation device**
Hochfrequenzvorrichtung für chirurgische Eingriffe
Dispositif haute fréquence pour interventions chirurgicales

(30) Priority: 23.06.2006 JP 2006173269
(43) Date of publication of application: 26.12.2007
(73) Proprietor: Fujinon Corporation, Saitama-shi, Saitama (JP)
(72) Inventor: Oyatsu, Masayuki, Saitama-shi Saitama (JP)
(74) Representative: Höhfeld, Jochen

(56) References cited:
- WO-A-95/20360
- WO-A-03/061499
- US-A- 5 234 429
- US-A- 5 441 499

## Description

### Background of the Invention

### 1. Field of the Invention

The present invention relates to a high frequency operation device which can be inserted into an operation device insertion channel formed in an endoscope to perform operations such as an operation to dissect a lesion mucous membrane and an operation to separate the dissected lesion mucous membrane.

### 2. Description of the Related Art

When, in an endoscopic examination, a lesion portion such as a tumor is found in the mucous membrane portion of an esophagus, a stomach, a duodenum, a large intestine or the like within inner wall of the body cavity, there is performed an operation to excise or remove the lesion mucous membrane portion using a high frequency operation device. In the operation to remove the lesion mucous membrane portion, in the related art, there has been widely used Endoscopic Mucosal Resection (EMR) method using a high frequency snare. However, this EMR method is not capable of removing a large lesion portion at a time but needs several times of removing operations, and also there is a fear that the lesion portion can be left in part. To solve this problem, recently, there has been employed Endoscopic Submucosal Dissection (ESD) method. According to this ESD method, an operation is executed in two stages: that is, firstly, the periphery of the lesion portion of the mucous membrane is cut open and, next, the lesion mucous membrane including a portion of a mucous membrane lower layer is separated from a muscular layer. According to the ESD method, there is provided an advantage that, even for a large lesion portion, the lesion mucous membrane portion can be removed completely by a single operation with no leftover.

A high frequency operation device used in the ESD method is structured such that a high frequency knife can be projected from and retreated into the leading end of a flexible sheath having an electric insulative property. Also, to the base end portion of the flexible sheath, there is connected an operation section; and, the flexible sheath can be inserted into an operation device insertion channel formed in an endoscope. As a high frequency operation device of this type, for example, there is known a high frequency operation device which is disclosed in JP-A-2004-313537. This known high frequency operation device is structured such that an operation wire is inserted into the interior portion of a flexible sheath, a knife portion serving as a high frequency operation section is connected to the leading end of the operation wire, and an operation section is connected to the base end portion of the flexible sheath. The operation section includes a slider connected to the main body shaft thereof, and the base end portion of the operation wire is connected to the slider; and, by pushing and pulling the slider along the main body shaft, the knife portion connected to the operation wire can be projected from and retreated into the leading end of the flexible sheath.

To form the knife portion, a plate-shaped electrode having a disk plate shape or a triangular plate shape is connected to the leading end of a stick-shaped electrode. And, there is also disclosed a knife portion in which a hook knife is formed by bending the leading end portion of a stick-shaped electrode. And, according to the disclosure of JP-A-2004-313537, by using an operation device including a knife portion with a plate-shaped electrode mounted thereon, it is possible to perform an operation to dissect a lesion mucous membrane and an operation to separate the thus dissected lesion mucous membrane. JP-A-2004-313537 further teaches that, when there is produced a bleeding portion while the operation is under execution, by pressing the plate-shaped electrode against the bleeding portion, the bleeding can be stopped.

When incising a lesion mucous membrane, it is necessary not to invade a muscular layer which is situated downwardly of a mucous membrane lower layer. For example, when a high frequency knife is contacted with the muscular layer in a state where a high frequency current flows in the high frequency knife, there is a possibility that there can be opened up a hole in the muscular layer and, as a result of this, there can occur excessive bleeding. When incising the mucous membrane, in order to separate the mucous membrane from the muscular layer, generally, a swelling solution such as a normal saline solution or a distention solution composed of hyaluronic acid is locally injected into the mucous membrane lower layer to swell the mucous membrane to be dissected. However, even when the mucous membrane is swollen by such local injection, there is a possibility that invasion of the high frequency knife into the muscular layer cannot be prevented. Since the high frequency knife is structured such that it can be projected from and retreated into the leading end of the flexible sheath, when the projecting length of the high frequency knife from the flexible sheath is regulated, the high frequency knife can be prevented from touching the muscular layer, thereby being able to secure the safety of the incising operation.

Here, referring to the regulation of the projecting length of the high frequency knife from the flexible sheath, while the maximum projection length is set equal to or longer than the thickness of the mucous membrane and equal to or smaller than the total thickness of the mucous membrane and mucous membrane lower layer, the high frequency knife is projected from the leading end of the flexible sheath and is pressed against the mucous membrane to dissect the lesion mucous membrane. In this case, the high frequency knife can be held for the leading end thereof not to reach the muscular layer.

When separating and removing the lesion mucous membrane, after the mucous membrane is dissected, the thus dissected mucous membrane must be separated. This mucous membrane separation operation is carried out by sliding the high frequency knife into the mucous membrane lower layer and cutting fibrous substances which constitute the mucous membrane lower layer. In this operation, while keeping the high frequency knife substantially parallel to the mucous membrane, it is operated such that it is swung right and left. Therefore, to carry out this mucous membrane separation operation efficiently, preferably, the projecting length of the high frequency knife from the flexible sheath may be set long to some extent.

In short, when the lesion mucous membrane is dissected and separated according to the ESD method, preferably, for the mucous membrane dissection operation, the projecting length of the high frequency knife from the flexible sheath may be limited and, for the mucous membrane separation operation, the projecting length of the high frequency knife may be set long. However, the high frequency operation device disclosed in JP-A-2004-313537 does not include a mechanism which can change the projecting length of the high frequency knife from the flexible sheath according to the kinds of the operations.

The same caveat applies to US 5,441,599, which describes a high-frequency operation device with two electrode members that are arranged in a telescopic manner and which are attached to respective drive members.

### Summary of the Invention

The present invention aims at solving the above problems found in the related-art high frequency operation device. Thus, it is an object of the invention to provide a high frequency operation device which, when carrying out the two kinds of operations, that is, the incising operation and separating operation, can adjust the projecting length of a high frequency operation section from the flexible sheath to the optimum length according to the respective operations.

In attaining the above object, according to the invention, there is provided a high frequency operation device comprising: a flexible sheath of an electric insulation member, the flexible sheath being insertable into an operation device insertion channel in an endoscope; a high frequency knife mounted on the flexible sheath; and an operation section connected to the flexible sheath to project the high frequency knife from a leading end of the flexible sheath, wherein the high frequency knife comprises first and second electrode members incorporated in a telescopic manner; wherein the operation section comprises: first and second drive members respectively that moves the first and second electrode members; and a connecting member that links together the first and second drive members and removes linkage between the first and second drive members, and wherein the high frequency operation device comprises: a first stage regulation portion that regulates a projecting length of the second electrode member, the first stage regulation portion being formed at the flexible sheath; and a second stage regulation portion that regulates a projecting length of the first electrode member from a leading end of the second electrode member, the second stage regulation portion being formed at the second electrode member.

When the present high frequency operation device is used to perform the operation to dissect the lesion mucous membrane portion and the operation to separate the dissected lesion mucous membrane portion, as regards the operation to dissect the lesion mucous membrane portion using the high frequency knife disposed on the leading end portion of the flexible sheath, the dissection operation is an operation to cut into the body tissues using the high frequency knife. Therefore, from the viewpoint of the operation efficiency of the dissection operation, preferably, the high frequency knife may be structured as a stick-shaped electrode. And, it is also possible to use other knives having proper structures such as a hook knife which can be made by bending the leading end portion of a stick-shaped electrode or an IT knife including an electric insulation member mounted on the leading end portion thereof. Also, the separation operation is an operation to swing the high frequency knife right and left. Therefore, in this operation, preferably, there may be used a straight-shaped knife or a stick-shaped electrode with the leading end portion thereof curved.

In order to cut open the mucous membrane, the projecting length of the high frequency knife from the leading end of the flexible sheath must be equal to or larger than the thickness of the mucous membrane layer. And, in order to prevent the leading end of the high frequency knife from touching the muscular layer, the projecting length must be equal to or smaller than the total thickness of the mucous membrane layer and mucous membrane lower layer. This is a first stage stroke and thus the first and second electrode members, which constitute the high frequency knife formed into a telescopic manner, can be projected from the leading end of the flexible sheath by an amount corresponding to the thus set projecting length while they are held at the reduced state thereof. Also, when separating the mucous membrane layer, the high frequency knife is directed substantially parallel to the mucous membrane layer and muscular layer; and, therefore, a second stage stroke is added to the first stage stroke, whereby the high frequency knife is projected further longer. What is projected at the then time is the second electrode member, whereas the first electrode member is held at the first stage stroke end position. Thanks to this, the high frequency knife can be projected from the leading end of the flexible sheath by an amount proper for efficient execution of the mucous membrane separation operation.

The first stage stroke is used to link together the first and second drive members, while the second stage stroke is used to project the first electrode member further from the first stage stroke end position. The linking member for linking together the first and second drive members and for removing such linkage, preferably, may be made of, for example, a set screw or a pin, or may be formed as a snap action mechanism. That is, it may preferably be structured such that it can link together the first and second drive members and can remove such linkage without using a tool.

The first drive member and first electrode member can be connected together using a first transmission member made of a flexible wire or the like. On the other hand, the second drive member and second electrode member can be connected together using a second transmission member. The second transmission member can be made of a wire which is arranged parallel to the first transmission member; however, preferably, the first transmission member may be made of a wire, the second transmission member may be made of a tube or a coil, and the wire may be inserted into the tube or coil. And, at least one of the first and second electrode members is or both of them are made of a conductive member, and one of them is or both of them are electrically connected to a high frequency power supply. Since the first and second electrode members are connected together in a telescopic manner and are thus contacted with each other, one of the first and second transmission members may be connected to the high frequency power supply, or the transmission members may be used only to transmit a drive force and the power may be supplied through a cable which is provided separately.

Although the first stage and second stage regulation portions may be disposed on the operation section side, in order to be able to move by all means the high frequency knife up to a given position regardless of the state of the flexible state, preferably, the projecting amount at least in the first stage may be regulated on the leading end side of the flexible sheath. Also, preferably, the projecting amount between the telescopically connected first and second electrode members may be also be regulated on the leading end side of the flexible sheath. The first stage regulation portion can be composed of a stopper ring which is mounted on the leading end of the flexible sheath; and, the second stage regulation portion can be structured such that the first electrode member is contacted with the base end portion of the second electrode member.

### Brief Description of the Drawings

Fig. 1 is a structure view of the whole of a high frequency operation device according to an embodiment of the invention;
Fig. 2 is a section view of the leading end portion of the high frequency operation device shown in Fig. 1;
Fig. 3 is a front view of the operation section side of the high frequency operation device;
Fig. 4 is a section view of the main body shaft of the operation section of the high frequency operation device in the slit direction thereof;
Fig. 5 is an enlarged section view taken along the portion X-X shown in Fig. 4;
Fig. 6 is an external view of a high frequency operation device according to an embodiment of the invention, showing a state where it is guided out from an operation device insertion channel formed in an endoscope;

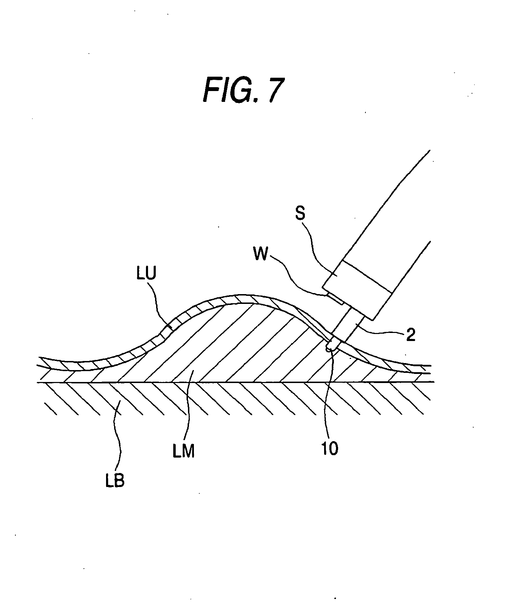

Fig. 7 is a section view of the tissues of the human body, showing a state where a dissection operation is being performed using the high frequency operation device; and

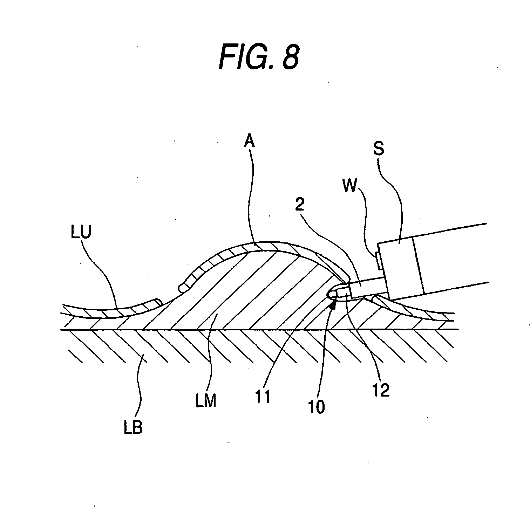

Fig. 8 is a section view of the tissues, showing a state where a mucous membrane separation operation is being performed.

### Detailed Description of the Invention

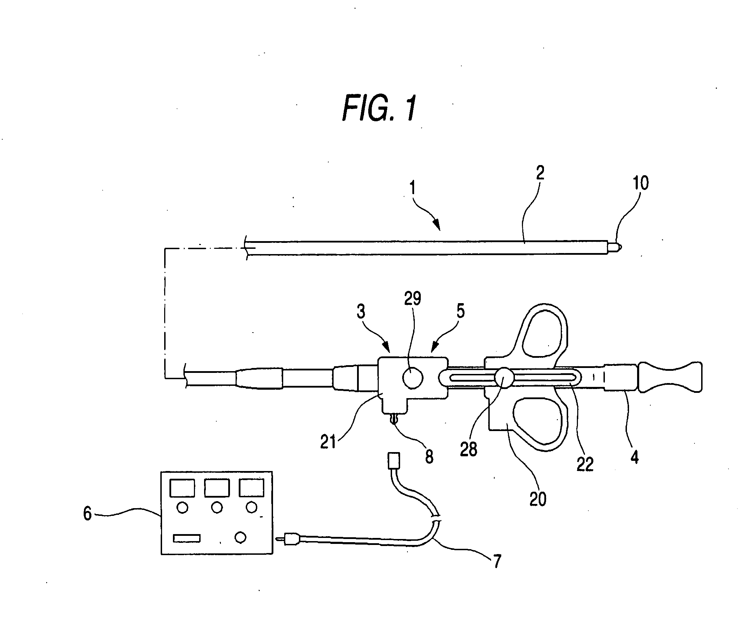

Now, description will be given below of a preferred embodiment according to the invention with reference to the accompanying drawings. In the present embodiment, description will be given of a high frequency operation device which is used to dissect and separate a lesion mucous membrane. By the way, it goes without saying that the high frequency operation device of the invention can also be used to carry out other kinds of operations. Fig. 1 shows the whole structure of the present high frequency operation device.

In Fig. 1, reference numeral 1 designates a high frequency operation device. The present high frequency operation device 1 includes a flexible sheath 2 which is made of a long insulation tube, while the base end portion of the flexible sheath 2 is connected to an operation section 3. The operation section 3 is composed of a main body shaft 4 and a slider 5 which is fitted with the main body shaft 4, can be slided in the axial direction of the main body shaft 4 and can be operated with the fingers of an operator. Reference numeral 6 stands for a high frequency power supply 6; and, to the high frequency power supply 6, there is removably connected a cable 7, while the cable 7 can be removably connected to a terminal part 8 which is mounted on the slider 5.

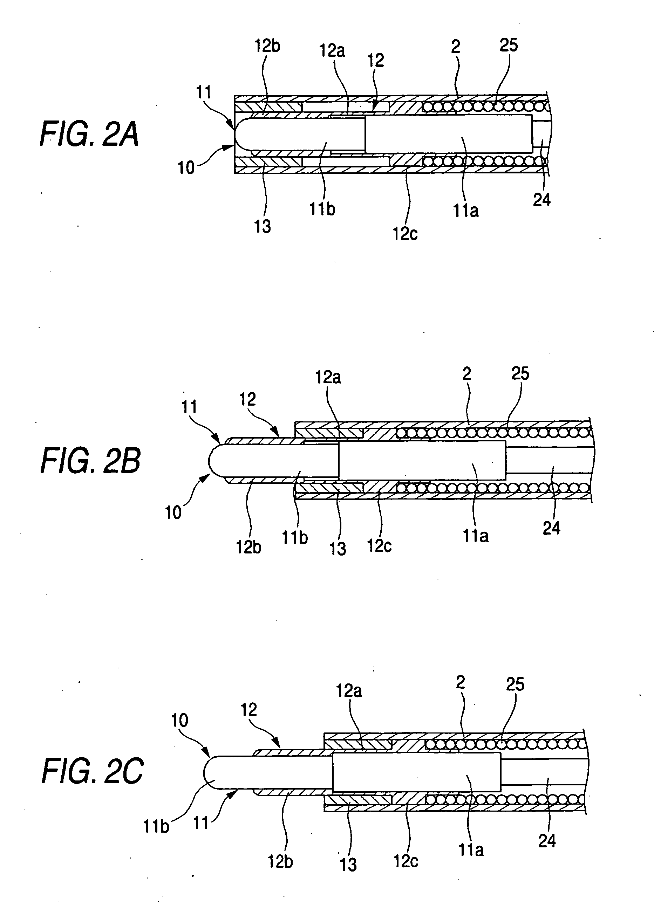

On the leading end portion of the flexible sheath 2, as shown in Fig. 2, there is mounted a high frequency knife 10. The high frequency knife 10 is composed of a stick-shaped electrode member 11 having a semi-spherical-shaped leading end portion, and a cylindrical-shaped second electrode member 12 which is fitted with the first electrode member 11. The first electrode member 11 is structured such that the base end side thereof is formed as a large diameter portion 11a and the leading end side thereof is formed as a small diameter portion 11b. Also, the cylindrical-shaped second electrode member 12 is fitted with the first electrode member 11 in such a manner that it can be slided, while the inner surface side thereof has a stepped structure. In other words, the second electrode member 12 is structured such that the base end side thereof is formed as a small thickness portion 12a which can be slided together with the large diameter portion 11a of the first electrode member 11, while the leading end side thereof is formed as a large thickness portion 12b which can be slided with respect to the small diameter portion 11b. And, these first and second electrode members 11 and 12 can be switched in shape substantially between a reduced state, in which only the semi-spherical surface portion of the first electrode member 11 is projected from the second electrode member 12, and an extended state in which the first electrode member 11 is greatly projected from the second electrode member 12.

Into the inner surface of the leading end portion of the flexible sheath 2, there is inserted a stopper ring 13 made of a pipe-shaped member which is formed of heat-resistant hard electrical insulation material such as ceramic, while the stopper ring 13 is fixed to the above-mentioned inner surface by bonding or the like. The inside diameter of the stopper ring 13 is set slightly smaller than the outside diameter of the second electrode member 12 and, therefore, the second electrode member 12 is slidably inserted into the stopper ring 13. And, the most base-side end portion of the second electrode member 12 is formed as an enlarged diameter portion 12c; and, the enlarged diameter portion 12c has a dimension larger than the inside diameter of the stopper ring 13.

According to the above structure, while no operation is being carried out, the high frequency knife 10, as shown in Fig. 2A, is held at its retreat position where the high frequency knife 10 is stored within the flexible sheath 2. As shown in Fig. 2B, when the first and second electrode members 11 and 12 are moved from this state to project integrally from the flexible sheath 2 while they are held at the reduced state, the enlarged diameter portion 12c of the second electrode member 12 is contacted with the base end face of the stopper ring 13 and the high frequency knife 10 is thereby turned into a dissection operation position. In this state, the second electrode member 12 provides the most projecting position thereof and, therefore, the enlarged diameter portion 12c of the second electrode member 12 and the end face of the stopper ring 13 cooperate together in constituting a first state regulation portion. Here, in this reduced state, there intervenes a given clearance between the large thickness portion 12b of the second electrode member 12 and the large diameter portion 11a of the first electrode member 11.

The first electrode member 11, as shown in Fig. 2C, can be moved until the second electrode member 12 is projected most and the large diameter portion 11a is contacted with a stepped portion provided by the large thickness portion 12b of the second electrode member 12, whereby the first electrode member 11 provides the extended state where it is projected most from the flexible sheath 2. That is, the state, where the first electrode member 11 is projected most from the second electrode member 12, provides the separation operation position of the high frequency knife 10. At the then time, the stepped portion provided by the large thickness portion 12b of the second electrode member 12 is contacted with the stepped portion provided by the large diameter portion 11a formed in the first electrode member 11, thereby providing a second stage regulation portion.

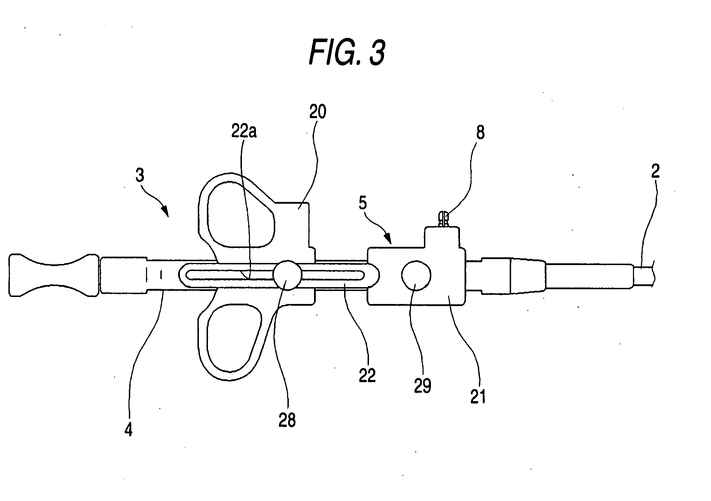

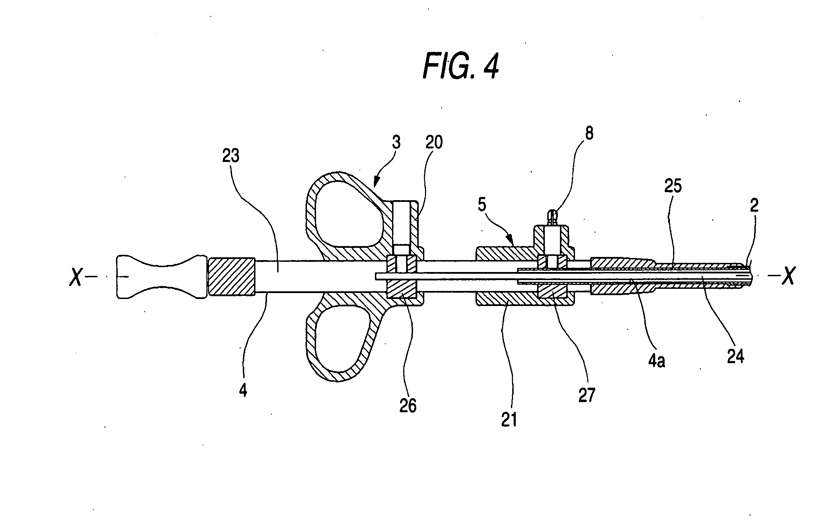

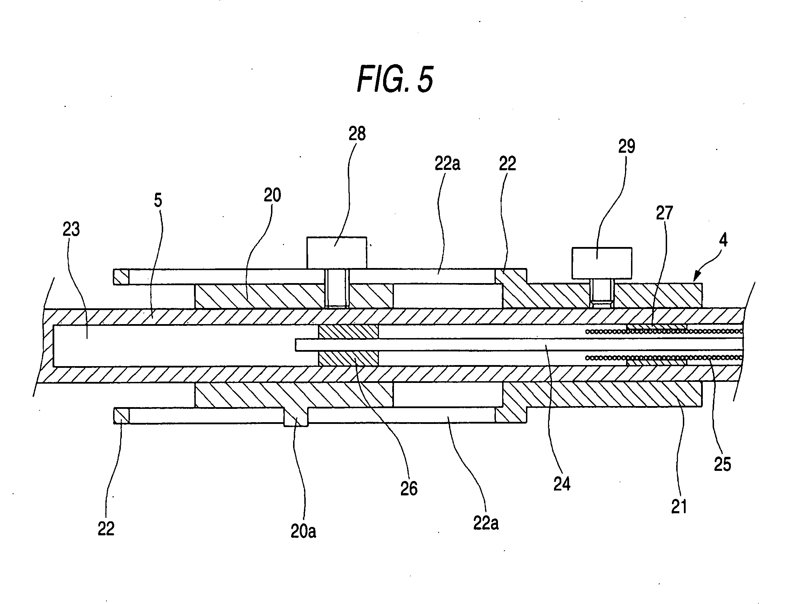

In this manner, the high frequency knife 10, which is composed of the first and second electrode members 11 and 12, is structured such that it can be projected in the two stages. And, in order to operate the high frequency knife 10 so as to project in the two stages, the operation section 3 is structured as shown in Figs. 3 to 5. That is, the slider 5, which is slidably mounted on the main body shaft 4, comprises a first drive part 20, a second drive part 21, and a linking member 22 which connects together the first and second drive parts 21 and 22 in such a manner that they can link to each other and also their mutual linkage can be removed. And, as shown in Figs. 4 and 5, in the main body shaft 4, there is formed a slit 23 which extends substantially over the entire length of the main body shaft 4; and, not only a wire 24, which functions as a first transmission member, inserted into the flexible sheath 2 and connected to the first electrode member 11 but also a coil 25 functioning as a second transmission member which is connected to the second electrode member 12 and through which the wire 24 is inserted, are extended into the slit 23 through a passage portion 4a opened up in the main body shaft 4.

Within the slit 23, there are further disposed a first sliding piece 26 connected to the first drive part 20 and a second sliding piece 27 connected to the second drive part 21. To the second sliding piece 27, there is connected the coil 25, while the wire 24 is guided out from the end portion of the coil 25 and the end portion of the wire 24 is connected to the first sliding piece 26. And, the terminal part 8, in the slider 5, is mounted on the second drive part 21 thereof, while the terminal part 8 is electrically connected to the coil 25. Therefore, the coil 25 is made of a coil-shaped metal wire rod which can conduct electricity. Also, at least the second sliding piece 27 is also made of a conductive member. And, to the terminal part 8, there are electrically connected not only the second electrode member 12 through the coil 25 but also the first electrode member 11 which is contacted with the second electrode member 12 in such a manner it is slidable with respect to the second electrode member 12.

Here, the first drive part 20 is connected to the second drive part 21 through the linking member 22 and, to serve this purpose, the connecting member 22 is made of a long plate-shaped member having an elongated bore 22a, while the leading end portion of the connecting member 22 is connected continuously with the second drive part 21. And, into the elongated bore 22a, there is inserted a connecting screw 28, while the connecting screw 28 is threadedly inserted into the first drive part 20. Therefore, when the connecting screw 28 is tightened, the first and second drive parts 20 and 21 are connected together; and, when the connecting screw 28 is loosened, the first drive part 20 can be slided alone along the main body shaft 4. Here, the linking member 22 having the elongated bore 22a is disposed on each side across the main body shaft 4. Specifically, the connecting screw 28 is mounted on one side linking member 22; and, into the elongated bore 22a of the other side linking member 22, there is engaged a projection 20a which is provided on the first drive part 20. Further, into the second drive part 21, there is threadedly inserted a set screw 29. Therefore, when the set screw 29 is tightened, the leading end portion thereof is pressed against the main body shaft 4, with the result that the second drive part 21 can be fixedly held on the main body shaft 4.

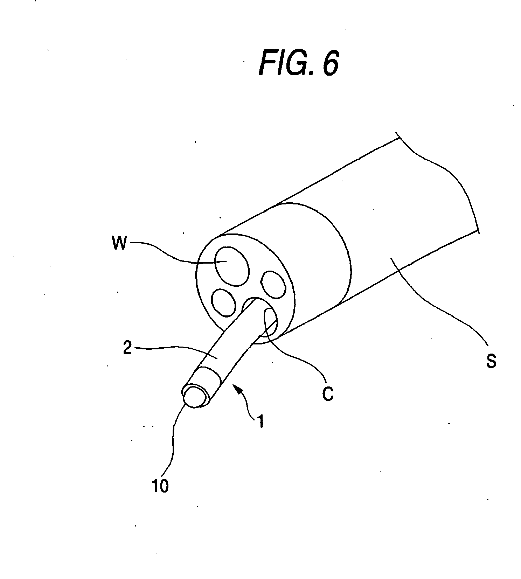

The high frequency operation device 1 having the above structure, as shown in Fig. 6, is inserted into the body cavity of the human body through an operation device insertion channel C formed in an endoscope insertion part S including an observation portion W and, when a lesion mucous membrane is found existing on the inner wall of the body cavity such as esophagus, stomach, duodenum, and large intestine, the high frequency operation device 1 is used to perform an operation to separate and remove such lesion mucous membrane portion. Now, description will be given below of an example of the operation to excise the lesion mucous membrane. This excise operation is carried out, for example, when, as the result of examination using an endoscope S, the existence of a lesion portion in the mucous membrane is confirmed. Also, this operation is carried out in two stages, that is, in one stage where the mucous membrane is dissected and in the other state where it is separated.

Firstly, to dissect the mucous membrane, the operation section 3 of the high frequency operation device 1 is operated, that is, the first and second drive parts 20 and 21 of the slider 5 are turned by the linking member 22 into such a state that they can be operated in linking with each other, and they are moved from the retreat position up to the dissection operation position. In other words, not only the first and second drive parts 20 and 21 are connected together through the linking member 22 by operating the connecting screw 28, but also the set screw 29 is loosened. As a result of this, the whole of the slider 5 can be slided along the main body shaft 4.

By operating the slider 5, as shown in Fig. 2B, the first and second electrode members 11 and 12, which constitute the high frequency knife 10, are projected a given length from the leading end of the flexible sheath 2 in the reduced state where only the semi-spherical surface portion of the first electrode member 11 is projected from the second electrode member 12. The projecting length of the high frequency knife 10 from the flexible sheath 2 at the then time is set larger than the thickness of a mucous membrane layer LU and smaller than the total thickness of the mucous membrane LU and mucous membrane lower layer LM. In this state, when power is supplied from a high frequency power supply 6, a high frequency current is allowed to flow in the high frequency knife 10, so that the mucous membrane layer LU can be cut open.

On the leading end portion of the flexible sheath 2, there is mounted the stopper ring 13, while the stopper ring 13 is disposed at the same position as the leading end face of the flexible sheath 2. Therefore, the contact area of the leading end of the flexible sheath 2 with the mucous membrane layer LU is large and, when the leading end face of the flexible sheath 2 is pressed lightly against the mucous membrane layer LU, the leading end face can be kept from pushing the mucous membrane layer LU. Also, the first stage regulation portion for regulating the projecting length of the high frequency knife 10 at the dissection operation position is interposed between the enlarged diameter portion 12c of the second electrode member 12 and the end face of the stopper ring 13, that is, it is disposed in the leading end portion of the flexible sheath 2. Therefore, the projecting length from the leading end of the flexible sheath 2 can be adjusted very accurately. By operating the high frequency knife 10 in this manner, not only the mucous membrane layer LU can be dissected positively but also the high frequency knife 10 can be prevented from reaching a position where it could possibly touch a muscular layer LB existing below of the mucous membrane lower layer LM, and thus the high frequency knife 10 can be prevented from injuring the muscular layer LB. And, when the mucous membrane lower layer LM is swollen by previously injecting a hyaluronic acid solution, a normal saline solution or the like into the mucous membrane lower layer LM locally, a safer operation can be realized.

The dissection operation is performed over the whole periphery of the lesion portion and, as a result of this, the mucous membrane layer LU existing in the periphery of the outer peripheral portion of the lesion mucous membrane is cut open and thus the mucous membrane lower layer LM is exposed. However, simply by cutting open the whole periphery of a lesion mucous membrane area D, the mucous membrane LU cannot be removed. That is, since the mucous membrane layer LU and muscular layer LB are connected together by the mucous membrane lower layer LM formed of fibers, it is necessary to cut the fibers and separate the mucous membrane layer LU from the muscular layer LB.

In this separation operation, while applying a high frequency current to the high frequency knife 10 from the high frequency power supply 6, the flexible sheath 2 is moved horizontally or is swung around to burn the mucous membrane lower layer LM due to the action of the high frequency current, thereby cutting the fibers. That is, the high frequency knife 10 is extended in a direction substantially parallel to the mucous membrane layer LU and muscular layer LB. Therefore, even when the high frequency knife 10 is projected fairly greatly from the leading end of the flexible sheath 2, there is no fear that the high frequency knife 10 can invade and injure the muscular layer LB; or rather, in order to perform the mucous membrane separation operation with efficiency, it is necessary to make longer the projecting length of the high frequency knife 10 from the flexible sheath 2.

As can be seen from the above description, in a state where the second drive part 21 constituting the slider 5 is set at the dissection operation position, by tightening the set screw 29, the second drive part 21 is fixed to the main body shaft 4. Also, by loosening the connecting screw 28, the linkage between the first and second drive parts 20 and 21 through the linking member 22 is removed. In this state, when the first drive part 20 is slided along the main body shaft 4, not only the second electrode member 12 is kept in a state where it is projected from the leading end of the flexible sheath 2, but also the first electrode member 11 is projected from the second electrode member 12 which shows a cylindrical shape. This provides a separation operation position in which the high frequency knife 10 is held in its extended state and is contacted with the second stage regulation portion composed of the step provided by the large thickness portion 12b of the second electrode member 12 and the step of the large diameter portion 11a of the first electrode member 11.

In this state, by swinging the high frequency knife 10, the mucous membrane lower layer LM can be separated. Here, since the second and first electrode members 12 and 11 are projected greatly from the leading end of the flexible sheath 2 and the high frequency current is applied to these two electrode members 12 and 11, the high frequency knife 10 can perform the mucous membrane separation operation with efficiency. And, the operation to swing the high frequency knife 10 can be carried out easily, for example, by curving the leading end portion of the endoscope insertion portion S. Thanks to this, the mucous membrane can be separated quickly and efficiently.

According to the invention, while the high frequency operation device is left inserted into the operation device insertion channel of the endoscope, two kinds of operations, that is, an operation to dissect a mucous membrane and an operation to separate thus dissected mucous membrane can be carried out safely and efficiently.

## Claims

1. A high frequency operation device (1) comprising:
a flexible sheath (2) of an electric insulation member, the flexible sheath being insertable into an operation device insertion channel in an endoscope;
a high frequency knife (10) mounted on the flexible sheath; and
an operation section (3) connected to the flexible sheath to project the high frequency knife from a leading end of the flexible sheath,
wherein the high frequency knife comprises first and second electrode members (11, 12) incorporated in a telescopic manner;
wherein the operation section comprises: first and second drive members (20, 21) respectively that moves the first and second electrode members; and a connecting member (22) that links together the first and second drive members and removes linkage between the first and second drive members, and
wherein the high frequency operation device comprises: a first stage regulation portion that regulates a projecting length of the second electrode member, the first stage regulation portion being formed at the flexible sheath; and a second stage regulation portion that regulates a projecting length of the first electrode member from a leading end of the second electrode member, the second stage regulation portion being formed at the second electrode member.

2. A high frequency operation device (1) as set forth in claim 1, further comprising:
a stopper ring (13) having a passage capable of guiding out the high frequency knife (10), the stopper ring being on an inner surface of the leading end of the flexible sheath (2),
wherein a base end portion (12c) of the second electrode member (12) is structured such that it can be connected with a base end portion of the stopper ring so as to form the first stage regulation portion and can be disconnected from a base end portion of the stopper ring.

3. A high frequency operation device (1) as set forth in claim 1, further comprising:
a first transmission member (24) that connects the first drive member (20) with the first electrode member (11); and
a second transmission member (25) that connects the second drive member (21) with the second electrode member (12),
wherein the first and second drive members are linked together by the connecting member (22) so that the first and second electrode members can be shifted up to a position of the first stage regulation portion; and
wherein the linkage between the first and second drive members by the connecting member is removed so that the first electrode member can be shifted up to a position of the second regulation portion by the first drive member.

## Patentansprüche

1. Hochfrequenz-Operationsvorrichtung (1) umfassend:
einen flexiblen Schaft (2) eines elektrischen Isoliergliedes, wobei der flexible Schaft in einen Operationsvorrichtungs-Einführkanal in einem Endoskop einführbar ist;
ein Hochfrequenz-Messer (10), welches an den flexiblen Schaft angebracht ist; und
ein Operationsteil (3), welcher mit dem flexiblen Schaft verbunden ist, um das Hochfrequenz-Messer aus einem vorderen Ende des flexiblen Schafts herauszustrecken,
wobei das Hochfrequenz-Messer erste und zweite Elektrodenglieder (11, 12) umfasst, welche nach Art eines Teleskops eingebaut sind;
wobei der Operationsteil umfasst: erste und zweite Antriebsglieder (20, 21), welche jeweils die ersten und zweiten Elektrodenglieder bewegen; und ein Verbindungsglied (22), welches die ersten und zweiten Antriebsglieder miteinander verbindet und die Verbindung zwischen den ersten und zweiten Antriebsgliedern löst, und
wobei die Hochfrequenz-Operationsvorrichtung umfasst: einen Regulierabschnitt einer ersten Stufe, der eine Herausstreckungslänge des zweiten Elektrodengliedes reguliert, wobei der Regulierabschnitt der ersten Stufe an dem flexiblen Schaft ausgebildet ist; und ein Regulierabschnitt einer zweiten Stufe, der eine Herausstreckungslänge des ersten Elektrodengliedes von einem vorderen Ende des zweiten Elektrodengliedes reguliert, wobei der Regulierabschnitt der zweiten Stufe an dem zweiten Elektrodenglied ausgebildet ist.

2. Hochfrequenz-Operationsvorrichtung (1) gemäß Anspruch 1 , weiterhin umfassend:
eine Stoppring (13) mit einem Durchlass, der das Hochfrequenz-Messer (10) herausführen kann, wobei sich der Stoppring an einer inneren Oberfläche des vorderen Endes des flexiblen Schafts (2) befindet,
wobei ein hinterer Endabschnitt (12c) des zweiten Elektrodengliedes (12) so strukturiert ist, dass er mit einem hinteren Endabschnitt des Stopprings verbunden werden kann, um den Regulierabschnitt der ersten Stufe auszubilden, und von einem hinteren Endabschnitt des Stopprings abgetrennt werden kann.

3. Hochfrequenz-Operationsvorrichtung (1) gemäß Anspruch 1, weiterhin umfassend:
ein erstes Transmissionsglied (24), welches das erste Antriebsglied (20) mit dem ersten Elektrodenglied (11) verbindet; und
ein zweites Transmissionsglied (25), welches das zweite Antriebsglied (21) mit dem zweiten Elektrodenglied (12) verbindet,
wobei die ersten und zweiten Antriebsglieder miteinander über das Verbindungsglied (22) verbunden sind, so dass die ersten und zweiten Elektrodenglieder bis zu einer Position des Regulierabschnitts der ersten Stufe verschoben werden können; und
wobei die Verbindung zwischen den ersten und zweiten Antriebsgliedern durch das Verbindungsglied gelöst wird, so dass das erste Elektrodenglied bis zu einer Position des zweiten Regulierabschnitts durch das erste Antriebsglied verschoben werden kann.

## Revendications

1. Dispositif d'opération haute fréquence (1), comprenant :
une gaine souple (2) d'un élément à isolation électrique, la gaine souple pouvant être introduite dans un canal d'introduction de dispositif d'opération dans un endoscope ;
un couteau haute fréquence (10) monté sur la gaine souple, et
une partie d'opération (3) reliée à la gaine souple pour projeter le couteau haute fréquence à partir d'une extrémité avant de la gaine souple,
dans lequel le couteau haute fréquence comprend des premier et second éléments formant électrodes (11,12) incorporés de manière télescopique ;
dans lequel la partie d'opération comprend : des premier et second éléments d'entraînement respectifs (20, 21) qui déplacent les premier et second éléments formant électrodes, et un élément de raccordement (22) qui relie ensemble les premier et second éléments d'entraînement et élimine la liaison entre les premier et second éléments d'entraînement, et
dans lequel le dispositif d'opération haute fréquence comprend : une partie de régulation de première étape qui régule une longueur de projection du second élément formant électrode, la partie de régulation de première étape étant formée au niveau de la gaine souple, et une partie de régulation de seconde étape qui régule une longueur de projection du premier élément formant électrode à partir d'une extrémité avant du second élément formant électrode, la partie de régulation de seconde étape étant formée au niveau du second élément formant électrode,

2. Dispositif d'opération haute fréquence selon la revendication 1, comprenant en outre:
un anneau de butée (13) présentant un passage pouvant guider la sortie du couteau haute fréquence (10), l'anneau de butée se trouvant sur une surface intérieure de l'extrémité avant de la gaine souple (2),
dans lequel une partie d'extrémité formant base (12c) du second élément formant électrode (12) est structurée de telle sorte qu'elle peut être raccordée à une partie d'extrémité formant base de l'anneau de butée afin de former la partie de régulation de première étape, et peut être désolidarisée d'une partie d'extrémité formant base de l'anneau de butée.

3. Dispositif d'opération haute fréquence selon la revendication 1, comprenant en outre:
un premier élément de transmission (24) qui relie le premier élément d'entraînement (20) au premier élément formant électrode (11), et
un second élément de transmission (25) qui relie le second élément d'entraînement (21) au second élément formant électrode (12),
dans lequel les premier et second éléments d'entraînement sont reliés ensemble par l'élément de raccordement (22), de telle sorte que les premier et second éléments formant électrodes puissent être déplacés jusqu'à une position de la partie de régulation de première étape, et
dans lequel la liaison entre les premier et second éléments d'entraînement par l'élément de raccordement est éliminée, de telle sorte que le premier élément formant électrode puisse être déplacé jusqu'à une position de la partie de régulation de seconde étape par le premier élément d'entraînement.
